# EUROPÄISCHE PATENTSCHRIFT

(11) **EP 2 501 667 B1**
(45) Veröffentlichungstag und Bekanntmachung des Hinweises auf die Patenterteilung: **02.11.2016**
(21) Anmeldenummer: 10776382.3
(22) Anmeldetag: 16.11.2010
(51) Int. Cl.: C07C 29/141, C07C 29/149, C07C 31/20, C07C 31/22, B01J 23/58, B01J 23/78, B01J 23/83, B01J 23/72, B01J 37/06

(54) **VERFAHREN ZUR HERSTELLUNG EINES GETRÄGERTEN HYDRIERKATALYSATORS MIT ERHÖHTER HYDRIERAKTIVITÄT**
METHOD FOR PRODUCING A SUPPORTED HYDROGENATION CATALYST HAVING INCREASED HYDROGENATION ACTIVITY
PROCÉDÉ DE PRODUCTION D'UN CATALYSEUR D'HYDROGÉNATION SUPPORTÉ PRÉSENTANT UNE ACTIVITÉ D'HYDROGÉNATION ACCRUE

(30) Priorität: 17.11.2009 EP 09176203
(43) Veröffentlichungstag der Anmeldung: 26.09.2012
(73) Patentinhaber: BASF SE, 67056 Ludwigshafen am Rhein (DE)
(72) Erfinder: TOMPERS, Rolf, 68259 Mannheim (DE); URTEL, Heiko, 67240 Bobenheim-Roxheim (DE); PINKOS, Rolf, 67098 Bad Dürkheim (DE); TEBBEN, Gerd-Dieter, 68239 Mannheim (DE); HEIMANN, Jens, 67549 Worms (DE); GUIXA GUARDIA, Maria, 68163 Mannheim (DE); BORCHERS, Sabine, 76872 Erlenbach bei Kandel (DE)
(86) Internationale Anmeldenummer: PCT/EP2010/067572
(87) Internationale Veröffentlichungsnummer: WO 2011/061185

(56) Entgegenhaltungen:
- EP-A1- 0 648 534
- EP-A1- 0 790 074
- EP-A1- 0 810 194
- EP-A1- 1 207 149
- EP-A2- 0 810 202
- WO-A1-2007/028411
- WO-A2-02/083818
- WO-A2-2010/089346
- DE-A1-102007 011 484
- US-A- 4 826 799

## Beschreibung

Die Erfindung betrifft ein Verfahren zur Herstellung eines geträgerten Hydrierkatalysators mit erhöhter Hydrieraktivität.

Die Hydrierung von Carbonylgruppen aufweisenden organischen Verbindungen an geträgerten Hydrierkatalysatoren ist an sich bekannt. So beschreibt die WO 2007/006719 einen Katalysator und ein Verfahren zur Hydrierung von Carbonylverbindungen. Es wird ein Katalysator eingesetzt, der durch Fällung eines Gemisches aus einer Kupfernitratlösung, einer Aluminiumnitratlösung und einer Lanthannitratlösung mit Natriumcarbonat hergestellt wird. Der gefällte Katalysator wird getrocknet und calciniert sowie kompaktiert. Das Kompaktat wird mit Kupferblättchen und Graphit gemischt und zu Tabletten verpresst. Diese Tabletten werden vor dem Einsatz in der Hydrierung mit Wasser oder Wasserdampf vorbehandelt. Es ist angegeben, dass die Behandlung bei einer Temperatur von 100 bis 140 °C und einem Druck von 1 bis 30 bar erfolgt, wobei das eingesetzte Wasser mit Hilfe von Mineralsäuren wie Salpetersäure, Schwefelsäure oder Salzsäure oder Natriumcarbonat oder Natronlauge auf einen pH-Wert von 4 bis 9, bevorzugt 6 bis 8,5 eingestellt werden kann.

Es wurde eine gesteigerte Hydrieraktivität und Selektivität des Katalysators unter Erhalt einer hohen Stabilität des Formkörpers gefunden.

Die Anfangsaktivität des Katalysators kann für einige Anwendungen jedoch noch verbesserungswürdig sein.

Aufgabe der vorliegenden Erfindung ist daher die Bereitstellung eines Verfahrens zur Herstellung eines geträgerten Hydrierkatalysators mit erhöhter Hydrieraktivität, ohne dabei die Selektivität oder die mechanische Stabilität des Trägerkatalysators zu verringern.

Die Aufgabe wird erfindungsgemäß gelöst durch ein Verfahren gemäß Anspruch 1 der anhängigen Ansprüche.

Es wurde erfindungsgemäß gefunden, dass gerade bei geträgerten Hydrierkatalysatoren, die Aluminiumoxid im Trägermaterial enthalten, durch eine Basenbehandlung bei einem pH-Wert von > 10 die Aktivität des Katalysators signifikant gesteigert werden kann, ohne die Selektivität und die mechanische Stabilität negativ zu beeinträchtigen. Dabei wird mit einer Basenlösung mit einem pH-Wert von > 10 behandelt bzw. bei einem pH-Wert von > 10 behandelt.

Der Begriff "geträgerter Hydrierkatalysator" umfasst Katalysatoren, die neben dem Hydriermetall oder den Hydriermetallen auch Trägermaterialien enthalten. Beispielsweise kann es sich um Katalysatoren handeln, in denen der Katalysatorträger durch Imprägnierung mit dem Hydriermetall behandelt wird. Ebenso sind Katalysatoren umfasst, die durch gemeinsame Fällung hergestellt werden, d. h. bei denen das Hydriermetall oder ein Salz oder Oxid davon zusammen mit dem Trägermaterial aus Vorläuferlösungen gemeinsam gefällt werden. Allgemein kann der Katalysator durch beliebige geeignete Verfahren erhalten werden, sofern ein Hydriermetall und ein Al₂O₃-haltiges Trägermaterial nebeneinander vorliegen. Bei den Katalysatoren kann es sich um Voll-, Tränk-, Schalen- oder Fällkatalysatoren handeln. Geeignete Katalysatoren sind beispielsweise in WO 2007/006719, US 2008/0299390, US 2008/0071120, US 7,510,591 EP1207149, WO2007/028411, US4826799, DE102007011 WO02/083818 und EP0790074 beschrieben.

Der erfindungsgemäß hergestellte geträgerte Hydrierkatalysator enthält ein Al₂O₃-haltiges Trägermaterial. Dabei enthält das Trägermaterial vorzugsweise mehr als 50 Gew.-%, besonders bevorzugt mehr als 75 Gew.-% Al₂O₃. Es kann sich um einen reinen Al₂O₃-Träger handeln oder um ein Al₂O₃-haltiges Trägermaterial das beispielsweise zusätzlich weitere Metalloxide wie Ceroxid, Titandioxid, Zirkonoxid, Siliziumdioxid und/oder Lanthanoxid enthält. Zudem kann der Träger neben Aluminium noch andere Metalle aufweisen. Vorzugsweise handelt es sich beim gegebenenfalls verbleibenden Anteil auch um Metalloxide.

Als Hydriermetall können alle üblichen in Hydrierkatalysatoren eingesetzten Aktivmetalle eingesetzt werden. Vorzugsweise ist das Hydriermetall ausgewählt aus den Gruppen 8 - 11 des Periodensystems der chemischen Elemente, besonders bevorzugt aus den Gruppen 10 und 11 des Periodensystems der chemischen Elemente. Besonders bevorzugt enthält das Hydriermetall Kupfer, Eisen, Nickel, Rhodium, Ruthenium, Palladium oder Platin oder ist aus diesen oder deren Gemischen ausgewählt.

Insbesondere für die Hydrierung von Carbonylverbindungen zu den entsprechenden Alkoholen werden technisch in breitem Umfang geträgerte Kupferkatalysatoren eingesetzt. Derartige Katalysatoren, die auch erfindungsgemäß verwendet werden können, enthalten die Aktivmasse auf dem Trägermaterial. Dabei kann die Aktivmasse nach einem beliebigen Verfahren auf ein vorhandenes Trägermaterial aufgebracht werden oder auch mit dem Trägermaterial ko-gefällt werden.

Im erfindungsgemäßen Herstellungsverfahren erfolgt die Basen-Behandlung des calcinierten geträgerten Hydrierkatalysators. Ein beispielsweise durch Ko-Fällung hergestellter Katalysator wird typischerweise nach der Fällung gewaschen, getrocknet und calciniert. Es können sich eine Tablettierung oder Extrusion sowie ein nochmaliges Calcinieren anschließen. Bei durch Imprägnierung hergestellten Katalysatoren erfolgt nach der Imprägnierung und Trocknung ebenfalls eine Calcinierung, worauf sich wiederum eine weitergehende Formgebung anschließen kann.

Das eingesetzte Katalysatormaterial wurde bei der vorausgehenden Herstellung bereits mindestens einem der genannten Calcinierungsschritte unterworfen. Es kann sich um bereits einer abschließenden Formgebung unterworfene Katalysatoren handeln, oder um nach der ersten Calcinierung erhaltene Katalysatorpulver.

Im erfindungsgemäßen Verfahren wird mit einer Basenlösung mit einem pH-Wert von > 10, vorzugsweise > 11, besonders bevorzugt > 12, speziell > 13 behandelt. Dabei weist die Basenlösung, die zur Behandlung eingesetzt wird, vorzugsweise den genannten pH-Wert auf, oder bei der Behandlung des Katalysators mit der Base liegt der genannte pH-Wert vor.

Erfindungsgemäß können beliebige geeignete Basenlösungen eingesetzt werden. Bevorzugt werden wässrige, alkoholische oder gemischt-wässrig/alkoholische Basenlösungen eingesetzt. Als Alkohole kommen beispielsweise Alkanole, vorzugsweise C₁₋₄-Alkanole wie Methanol, Ethanol, Propanol, Isopropanol oder die Butanole in Betracht. In wässrig-alkoholischen Lösungen beträgt der Alkoholanteil vorzugsweise maximal 30 Gew.-%, besonders bevorzugt maximal 20 Gew.-%, insbesondere maximal 10 Gew.-%. Besonders bevorzugt wird mit wässrigen Lösungen gearbeitet.

Die verwendete Base kann in einem weiten Bereich frei gewählt werden. Bevorzugt enthält die Basenlösung ein Alkalimetall- oder Erdalkalimetall-Hydroxid oder ein Gemisch davon. Besonders geeignete Alkalimetallhydroxide sind Natronlauge oder Kalilauge. Besonders geeignete Erdalkalimetallhydroxide sind Magnesiumhydroxid, Calciumhydroxid und Bariumhydroxid.

Besonders bevorzugt enthält die Basenlösung Natronlauge oder Kalilauge, insbesondere Natronlauge.

Besonders bevorzugt wird mit einer wässrigen Natronlauge behandelt.

Die Behandlung mit der Basenlösung erfolgt durch Besprühen des Katalysators mit der Basenlösung oder Eintauchen des Katalysators in eine Basenlösung, wobei der Katalysator gleichzeitig mechanisch bewegt werden kann. Bevorzugt wird mit einer Menge der Basenlösung gearbeitet, die zum vollständigen Bedecken des Katalysators ausreichend ist. Die Temperatur bei der Behandlung liegt dabei im Bereich von 20 bis 120 °C, vorzugsweise 35 bis 105 °C, insbesondere 50 bis 100 °C. In Abhängigkeit von der Basenkonzentration bzw. dem pH-Wert und der Temperatur kann die Behandlungszeit gewählt werden. Diese liegt im Bereich von 1 bis 300 Stunden, vorzugsweise 5 bis 200 Stunden, insbesondere 10 bis 150 Stunden.

Besonders bevorzugt wird mit wässriger 0,3- bis 3,0-molarer Natronlauge behandelt, besonders bevorzugt mit wässriger 0,5- bis 2,0-molarer Natronlauge. Dabei liegt der pH-Wert insbesondere oberhalb von 13,5 bzw. oberhalb von 14.

Durch die Behandlung mit der Basenlösung kann ein geringer Teil des im Träger enthaltenen Aluminiumoxids herausgelöst werden. Insbesondere im Randbereich des behandelten Formkörpers kann eine Abreicherung des Aluminiumoxids stattfinden. Durch die Behandlung mit der Basenlösung kann ebenfalls ein Teil des amorph oder röntgen-amorph vorliegenden Aluminiumoxids in feinkristallines Böhmit (AIOOH) umgewandelt werden. Diese strukturellen Veränderungen des Trägermaterials können der Ursprung der gesteigerten Aktivität sein.

Durch die Behandlung mit der Basenlösung kann sich der Aluminiumgehalt im Hydrierkatalysator und/oder im kalzinierten Katalysatorpulver um 0 bis 6 Gew.-%, bevorzugt 0,1 bis 6 Gew.-%, insbesondere 0,1 bis 3 Gew.-%, berechnet als elementares Aluminium, verringern.

Die Aktivitätssteigerung ist dabei nicht wie bei klassischen Raney-Metallsystemen auf das komplette Auslaugen des metallischen Aluminiums und die damit einhergehende Vergrößerung der Metalloberfläche zurückzuführen. In den erfindungsgemäß behandelten Katalysatoren wird nur ein deutlich geringerer Teil des Aluminiums, vorliegend als Aluminiumoxid, Aluminiumoxidhydroxid und/oder Aluminiumhydroxyd aus den Trägerkatalysatoren, beispielsweise Tabletten, gelöst. Die Oberfläche des Hydriermetalls ändert sich dabei in aller Regel nicht signifikant.

Besonders bevorzugt werden erfindungsgemäß Katalysatoren behandelt, die wie in WO 2007/006719 beschrieben hergestellt wurden.

Derartige Katalysatoren werden hergestellt in einem Verfahren, in dem
- ein oxidisches Material, umfassend Kupferoxid und Aluminiumoxid und mindestens eines der Oxyde des Eisens, Lanthans, Wolframs, Molybdäns, Titans, Zirkoniums, Zinns oder Mangans bereitgestellt wird,
- dem oxidischen Material pulverförmiges metallisches Kupfer, Kupferblättchen, pulverförmiger Zement, Graphit oder ein Gemisch davon zugegeben werden,
- das so resultierende Gemisch zu einem Formkörper verformt wird und
- der Formkörper nach dem vorliegenden erfindungsgemäßen Verfahren behandelt wird.

Besonders bevorzugt enthält das oxidische Material, bezogen auf das Gesamtgewicht des oxidischen Materials nach Calcinierung, - Kupferoxid mit einem Anteil im Bereich von 50 ≤ x ≤ 80, vorzugsweise 55 ≤ x ≤ 75 Gew.-%,
- Aluminiumoxid mit einem Anteil im Bereich von 15 ≤ y ≤ 35, vorzugsweise 20 ≤ y ≤ 30 Gew.-% und
- mindestens einer der Oxide des Eisens, Lanthans, Wolframs, Molybdäns, Titans, Zirkoniums, Zinns oder Mangans mit einem Anteil im Bereich von 1 ≤ z ≤ 30, bevorzugt 2 ≤ z ≤ 25 Gew.-%,
wobei gilt: 80 ≤ x + y + z ≤ 100, insbesondere 95 ≤ x + y + z ≤ 100, wobei Zement nicht dem oxidischen Material im obigen Sinne zugerechnet wird.

Der Anteil des zugegebenen metallischen Kupfers beträgt vorzugsweise 1 bis 40 Gew.-%, bezogen auf das Gesamtgewicht des oxidischen Materials.

Die Zugabe von Graphit erfolgt vorzugsweise in Mengen von 0,5 bis 5 Gew.-%, bezogen auf das Gesamtgewicht des oxidischen Materials.

Nachfolgend wird ein bevorzugtes erfindungsgemäßes Verfahren zur Herstellung des Ausgangskatalysators näher beschrieben.

Der in dem erfindungsgemäßen Verfahren verwendete Katalysator zeichnet sich dadurch aus, dass die Aktivkomponente Kupfer, die Komponente Aluminium und die Komponente mindestens eines der Oxide des Eisens, Lanthans, Wolframs, Molybdäns, Titans, Zirkoniums, Zinns oder Mangans bevorzugt mit einer Sodalösung simultan oder nacheinander gefällt werden, im Anschluss getrocknet, calciniert, tablettiert oder extrudiert und nochmals calciniert wird.

Insbesondere kommen folgende Fällungsmethoden in Betracht:
A) Eine Kupfersalzlösung, eine Aluminiumsalzlösung und eine Lösung eines Salzes des Eisens, Lanthans, Wolframs, Molybdäns, Titans, Zirkoniums, Zinn oder Mangans oder eine Lösung, enthaltend Kupfer-, Aluminium- und ein Salz des Eisens, Lanthans, Wolframs, Molybdäns, Titans, Zirkoniums, Zinn oder Mangans, wird parallel oder nacheinander mit einer Sodalösung gefällt. Das gefällte Material wird im Anschluss getrocknet und ggf. calciniert.
B) Fällung einer Kupfersalzlösung und einer Lösung eines Salzes des Eisens, Lanthans, Wolframs, Molybdäns, Titans, Zirkoniums, Zinns oder Mangans oder einer Lösung, enthaltend Kupfersalz und mindestens ein Salz des Eisens, auf einen vorgefertigten Aluminiumoxidträger. Dieser liegt in einer besonders bevorzugten Ausführungsform als Pulver in einer wässrigen Suspension vor. Das Trägermaterial kann aber auch als Kugeln, Stränge, Splitt oder Tabletten vorliegen.

B1) In einer Ausführungsform (I) wird eine Kupfersalzlösung und eine Lösung eines Salzes des Eisens, Lanthans, Wolframs, Molybdäns, Titans, Zirkoniums, Zinn oder Mangans oder eine Lösung, enthaltend Kupfersalz und ein Salz des Eisens, Lanthans, Wolframs, Molybdäns, Titans, Zirkoniums, Zinn oder Mangans, bevorzugt mit Sodalösung, gefällt. Als Vorlage wird eine wässrige Suspension des Trägermaterials Aluminiumoxid verwendet.

Ausgefällte Niederschläge, die aus A) oder B) resultieren, werden in üblicher Weise filtriert und vorzugsweise alkalifrei gewaschen, wie dies beispielsweise in der DE 198 09 418.3 beschrieben ist.

Sowohl die Endprodukte aus A) als auch die aus B) werden bei Temperaturen von 50 bis 150 °C, vorzugsweise bei 120 °C getrocknet und im Anschluss ggf. vorzugsweise 2 Stunden bei im Allgemeinen 200 bis 600 °C, insbesondere bei 300 bis 500 °C calciniert.

Als Ausgangssubstanzen für A) und/oder B) können prinzipiell alle in den bei der Aufbringung verwendeten Lösungsmitteln löslichen Cu(l) und/oder Cu(II)-Salze, wie beispielsweise Nitrate, Carbonate, Acetate, Oxalate oder Ammonium-Komplexe, analoge Aluminiumsalze und Salze des Eisens, Lanthans, Wolframs, Molybdäns, Titans, Zirkoniums, Zinn oder Mangans verwendet werden. Besonders bevorzugt für Verfahren gemäß A) und B) wird Kupfernitrat eingesetzt.

In dem erfindungsgemäßen Verfahren wird das oben beschriebene getrocknete und gegebenenfalls calcinierte Pulver bevorzugt zu Tabletten, Ringen, Ringtabletten, Extrudaten, Wabenkörpern oder ähnlichen Formkörpern verarbeitet. Hierfür sind sämtliche aus dem Stand der Technik geeigneten Verfahren denkbar. Besonders bevorzugt wird ein Katalysatorformkörper oder ein Katalysatorextrudat mit einem Durchmesser d und einer Höhe h < 5 mm, Katalysatorkugeln mit einem Durchmesser d < 6 mm oder Katalysator-Wabenkörper mit einem Zellendurchmesser r_{z} < 5 mm verwendet. Die Zusammensetzung des oxidischen Material ist im allgemeinen so beschaffen, dass der Anteil an Kupferoxid im Bereich von 40 bis 90 Gew.-%, der Anteil an Oxiden des Eisens, Lanthans, Wolframs, Molybdäns, Titans, Zirkoniums, Zinns oder Mangans im Bereich von 0 bis 50 Gew.-% und der Anteil an Aluminiumoxid im Bereich bis zu 50 Gew.-%, jeweils bezogen auf das Gesamtgewicht der Summe der oben genannten oxidischen Bestandteile, liegt, wobei diese drei Oxide zusammen mindestens 80 Gew.-% des oxidischen Materials nach Calcinierung darstellen, wobei Zement nicht dem oxidischen Material in obigem Sinne zugerechnet wird.

Nach der erfindungsgemäßen Behandlung mit der Basenlösung wird der Katalysator vorzugsweise erneut gewaschen, getrocknet und gegebenenfalls nochmals calciniert.

Der wie vorstehend beschriebene bevorzugte Katalysator kann noch näher beschrieben werden:
Im allgemeinen wird dem oxidischen Material pulverförmiges Kupfer, und/oder Kupferblättchen und optional zusätzlich pulverförmiger Zement oder Graphit oder ein Gemisch davon im Bereich von 0,5 bis 40 Gew.-%, bevorzugt im Bereich von 2 bis 20 Gew.-% und besonders bevorzugt im Bereich von 3 bis 18 Gew.-%, jeweils bezogen auf das Gesamtgewicht des oxidischen Materials, zugesetzt.

Als Zement wird vorzugsweise ein Tonerdezement eingesetzt. Besonders bevorzugt besteht der Tonerdezement im Wesentlichen aus Aluminiumoxid und Calciumoxid, und besonders bevorzugt besteht er aus ungefähr 75 bis 85 Gew.-% Aluminiumoxid und ungefähr 15 bis 25 Gew.-% Calciumoxid. Ferner kann ein Zement auf Basis Magnesiumoxid/Aluminiumoxid, Calciumoxid/Siliciumoxid und Calciumoxid/Aluminiumoxid/ Eisenoxid verwendet werden.

Insbesondere kann das oxidische Material in einem Anteil von höchstens 10 Gew.-%, bevorzugt höchstens 5 Gew.-%, bezogen auf das Gesamtgewicht des oxidischen Materials, mindestens eine weitere Komponente aufweisen, die ausgewählt wird aus der Gruppe bestehend aus den Elementen Re, Fe, Ru, Co, Rh, Ir, Ni, Pd und Pt.

In einer weiter bevorzugten Ausführungsform des erfindungsgemäßen Verfahrens wird dem oxidischen Material vor dem Verformen zum Formkörper zusätzlich zu dem Kupferpulver, und/oder den Kupferblättchen und dem optionalen Zementpulver bzw. dem Gemisch davon Graphit zugesetzt. Vorzugsweise wird soviel Graphit zugegeben, dass die Verformung zu einem Formkörper besser durchgeführt werden kann. In einer bevorzugten Ausführungsform werden 0,5 bis 5 Gew.-% Graphit, bezogen auf das Gesamtgewicht des oxidischen Materials, zugegeben. Dabei ist es gleichgültig, ob Graphit dem oxidischen Material vor oder nach oder gleichzeitig mit dem Kupferpulver, den Kupferblättchen oder dem Zementpulver oder dem Gemisch davon zugesetzt wird.

Nach Zugabe des Kupferpulvers, und/oder der Kupferblättchen und optional des Zementpulvers oder des Gemischs davon und gegebenenfalls Graphit zu dem oxidischen Material wird der im Anschluss an die Verformung erhaltene Formkörper gegebenenfalls mindestens einmal calciniert über eine Zeit von im allgemeinen 0,5 bis 10 h, bevorzugt 0,5 bis 2 Stunden. Die Temperatur bei diesem mindestens einen Calcinierschritt liegt im Allgemeinen im Bereich von 200 bis 600 °C, bevorzugt im Bereich von 250 bis 500 °C und besonders bevorzugt im Bereich von 270 bis 400 °C.

Im Falle der Formgebung mit Zementpulver kann es vorteilhaft sein, den vor der Calcinierung erhaltenen Formkörper mit Wasser zu befeuchten und anschließend zu trocknen.

Bei Einsatz als Katalysator in der oxidischen Form wird der Formkörper vor Beschickung mit der Hydrierlösung mit reduzierenden Gasen, beispielsweise Wasserstoff, vorzugsweise Wasserstoff/Inertgasgemischen, insbesondere Wasserstoff/Stickstoffgemischen bei Temperaturen im Bereich von 20 bis 500 °C, bevorzugt im Bereich von 150 bis 350 °C und insbesondere im Bereich von 180 bis 200 °C vorreduziert. Bevorzugt wird dabei ein Gemisch mit einem Wasserstoffanteil im Bereich von 1 bis 100 Vol.-%, besonders bevorzugt im Bereich von 1 bis 50 Vol.-% verwendet.

In einer bevorzugten Ausführungsform wird der Formkörper vor dem Einsatz als Katalysator in an sich bekannter Weise durch Behandlung mit reduzierenden Medien aktiviert. Das Aktivieren erfolgt entweder vorab in einem Reduktionsofen oder nach dem Einbau im Reaktor. Ist der Katalysator vorab im Reduktionsofen aktiviert worden, wird er in den Reaktor eingebaut und direkt unter Wasserstoffdruck mit der Hydrierlösung beschickt. Je nach Hydriermetall wie zum Beispiel im Falle von Cu oder Ni ist der aktivierte Katalysator stark pyrophor. Ein direkter Kontakt des trocknen aktivierten Katalysators mit Luft führt demnach leicht zur Beschädigung des Katalysators sowie zu einer ernsthaften Gefährdung der Sicherheit. Wurde der Katalysator zuvor in einem Reduktionsofen aktiviert, empfiehlt es sich daher, den aktivierten Katalysator für den Transport mit einem inerten Lösungsmittel zu überschichten und anschließend lösungsmittelfeucht in den Reaktor einzubauen. Hierfür eignen sich zum Beispiel Wasser, Alkohole oder mäßig- bis schwerflüchtige Alkane. Besonders vorteilhaft kann der Transport zum Reaktor in einem dem späteren Prozess intrinsischen Stoff wie zum Beispiel dem Edukt (oft eine Carbonylverbindung) oder dem Produkt (oft ein Alkohol) erfolgen. Alternativ kann der aktivierte Katalysator auch unter einem Inertgas wie zum Beispiel Stickstoff oder Argon transportiert und in den Reaktor eingebaut werden.

Bevorzugtes Einsatzgebiet der nach dem erfindungsgemäßen Verfahren hergestellten Formkörper ist die Hydrierung von Carbonylgruppen aufweisenden organischen Verbindungen im Festbett. Andere Ausführungsformen wie beispielsweise die Wirbelreaktion mit in auf- und abwirbelnder Bewegung befindlichem Katalysatormaterial ist jedoch ebenfalls möglich. Die Hydrierung kann in der Gasphase oder in der Flüssigphase durchgeführt werden. Vorzugsweise wird die Hydrierung in flüssiger Phase durchgeführt, beispielsweise in Riesel- oder Sumpffahrweise. Bei Arbeiten in Rieselfahrweise lässt man das flüssige, die zu hydrierende Carbonylverbindung enthaltende Edukt in dem Reaktor, der unter Wasserstoffdruck steht, über das in diesem angeordnete Katalysatorbett rieseln, wobei sich auf dem Katalysator ein dünner Flüssigkeitsfilm ausbildet. Dagegen wird beim Arbeiten in Sumpffahrweise Wasserstoffgas in den mit der flüssigen Reaktionsmischung gefluteten Reaktor eingeleitet, wobei der Wasserstoff das Katalysatorbett in aufsteigenden Gasperlen passiert.

In einer Ausführungsform wird die zu hydrierende Lösung im geraden Durchgang über die Katalysatorschüttung gepumpt. In einer anderen Ausführungsform des erfindungsgemäßen Verfahrens wird ein Teil des Produkts nach Durchgang durch den Reaktor als Produktstrom kontinuierlich abgezogen und ggf. durch einen zweiten Reaktor, wie oben definiert, geleitet. Der andere Teil des Produkts wird zusammen mit frischem, die Carbonylverbindung enthaltendem Edukt dem Reaktor erneut zugeführt. Diese Verfahrensweise wird im Folgenden als Kreislauffahrweise bezeichnet.

Wird als Ausführungsform die Rieselfahrweise gewählt, ist hierbei die Kreislauffahrweise bevorzugt. Weiter bevorzugt wird in Kreislauffahrweise unter Verwendung eines Haupt- und Nachreaktors gearbeitet.

Das Verfahren eignet sich zur Hydrierung von Carbonylverbindungen wie z. B. Aldehyden und Ketonen, Carbonsäuren, Carbonsäureestern, Carbonsäureanhydriden oder Lactonen zu den entsprechenden Alkoholen, wobei aliphatische und cycloaliphatische gesättigte und ungesättigte Carbonylverbindungen bevorzugt sind. Bei aromatischen Carbonylverbindungen kann es zur Bildung unerwünschter Nebenprodukte durch Hydrierung des aromatischen Kerns kommen. Die Carbonylverbindungen können weitere funktionelle Gruppen wie Hydroxy- oder Aminogruppen tragen. Ungesättigte Carbonylverbindungen werden in der Regel zu den entsprechenden gesättigten Alkoholen hydriert. Der Begriff "Carbonylverbindungen", wie er im Rahmen der Erfindung verwendet wird, umfasst alle Verbindungen, die eine C=O-Gruppe aufweisen, einschließlich Carbonsäuren und deren Derivaten. Selbstverständlich können auch Gemische aus zwei oder mehr als zwei Carbonylverbindungen gemeinsam hydriert werden. Ferner kann auch die einzelne zu hydrierende Carbonylverbindung mehr als eine Carbonylgruppe enthalten.

Bevorzugt wird das Verfahren zur Hydrierung aliphatischer Aldehyde, Hydroxyaldehyde, Ketone, Säuren, Ester, Anhydride, Lactone und Zucker eingesetzt.

Bevorzugte aliphatische Aldehyde sind verzweigte und unverzweigte gesättigte und/oder ungesättigte aliphatische C₂-C₃₀-Aldehyde, wie sie beispielsweise durch Oxosynthese aus linearen oder verzweigten Olefinen mit interner oder terminaler Doppelbindung erhältlich sind. Ferner können auch oligomere Verbindungen, die auch mehr als 30 Carbonylgruppen enthalten, hydriert werden.

Als Beispiel für aliphatische Aldehyde sind zu nennen:
Formaldehyd, Propionaldehyd, n-Butyraldehyd, iso-Butyraldehyd, Valeraldehyd, 2Methylbutyraldehyd, 3-Methylbutyraldehyd (Isovaleraldehyd), 2,2-Dimethylpropionaldehyd (Pivalinaldehyd), Capronaldehyd, 2-Methylvaleraldehyd, 3-Methylvaleraldehyd, 4-Methylvaleraldehyd, 2-Ethylbutyraldehyd, 2,2-Dimethylbutyraldehyd, 3,3-Dimethyl-butyraldehyd, Caprylaldehyd, Caprinaldehyd, Glutardialdehyd.

Neben den genannten kurzkettigen Aldehyden sind insbesondere auch langkettige aliphatische Aldehyde geeignet, wie sie beispielsweise durch Oxosynthese aus linearen α-Olefinen erhalten werden können.

Besonders bevorzugt sind Enalisierungsprodukte, wie z. B. 2-Ethylhexenal, 2-Methylpentenal, 2,4-Diethyloctenal oder 2,4-Dimethylheptenal.

Bevorzugte Hydroxyaldehyde sind C₃-C₁₂-Hydroxyaldehyde, wie sie beispielsweise durch Aldolreaktion aus aliphatischen und cycloaliphatischen Aldehyden und Ketonen mit sich selbst oder Formaldehyd zugänglich sind. Beispiele sind 3-Hydroxypropanal, Dimethylolethanal, Trimethylolethanal (Pentaerythrital), 3-Hydroxybutanal (Acetaldol), 3-Hydroxy-2-ethylhexanal (Butylaldol), 3-Hydroxy-2-methylpentanal (Propienaldol), 2-Methylolpropanal, 2,2-Dimethylolpropanal, 3-Hydroxy-2-methylbutanal, 3-Hydroxypentanal, 2-Methylolbutanal, 2,2-Dimethylolbutanal (DMB), Hydroxypivalinaldehyd. Besonders bevorzugt sind Hydroxypivalinaldehyd (HPA) und Dimethylolbutanal (DMB).

Bevorzugte Ketone sind Aceton, Butanon, 2-Pentanon, 3-Pentanon, 2-Hexanon, 3-Hexanon, Cyclohexanon, Isophoron, Methylisobutylketon, Mesityloxid, Acetophenon, Propiophenon, Benzophenon, Benzalaceton, Dibenzalaceton, Benzalacetophenon, 2,3-Butandion, 2,4-Pentandion, 2,5-Hexandion und Methylvinylketon.

Darüber hinaus können Carbonsäuren und Derivate davon, vorzugsweise solche mit 1-20 C-Atomen umgesetzt werden. Insbesondere sind die folgenden zu nennen:
Carbonsäuren, wie z. B. Ameisensäure, Essigsäure, Propionsäure, Buttersäure, Isobuttersäure, n-Valeriansäure, Trimethylessigsäure ("Pivalinsäure"), Capronsäure, Önanthsäure, Caprylsäure, Caprinsäure, Laurinsäure, Myristinsäure, Palmitinsäure, Stearinsäure, Acrylsäure, Methacrylsäure, Ölsäure, Elaidinsäure, Linolsäure, Linolensäure, Cyclohexancarbonsäure, Benzoesäure, Phenylessigsäure, o-Toluylsäure, m-Toluylsäure, p-Toluylsäure, o-Chlorbenzoesäure, p-Chlorbenzoesäure, o-Nitrobenzoesäure, p-Nitrobenzoesäure, Salicylsäure, p-Hydroxybenzoesäure, Anthranilsäure, p-Aminobenzoesäure, Oxalsäure, Malonsäure, Bernsteinsäure, Glutarsäure, Adipinsäure, Pimelinsäure, Korksäure, Azelainsäure, Sebacinsäure, Maleinsäure, Fumarsäure, Phthalsäure, Isophthalsäure, Terephthalsäure;
Carbonsäureester, wie z. B. die C₁-C₁₂-Alkylester der oben genannten Carbonsäuren, insbesondere Methylformiat, Essigester, Buttersäurebutylester, Phthalsäure-, IsoPhthalsäure-, Terephthalsäure-, Adipinsäure-, Maleinsäuredialkylester wie z. B. die Dimethylester dieser Säuren, (Meth)acrylsäuremethylester, Butyrolacton, Caproiacton und Polycarbonsäureester, wie z. B. Polyacrylund Polymethacrylsäureester und deren Copolymere und Polyester, wie z. B. Polymethylmethacrylat, Terephthalsäureester und andere technische Kunststoffe, wobei hier insbesondere Hydrogenolysen, also die Umsetzung von Estern zu den entsprechenden Säuren und Alkoholen, durchgeführt werden; Fette;
Carbonsäureanhydride, wie z. B. die Anhydride der oben genannten Carbonsäuren, insbesondere Essigsäureanhydrid, Propionsäureanhydrid, Benzoesäureanhydrid und Maleinsäureanhydrid;
Carbonsäureamide, wie z. B. Formamid, Acetamid, Propionamid, Stearamid, Terephthalsäureamid.

Ferner können auch Hydroxycarbonsäuren, wie z. B. Milch-, Äpfel-, Wein- oder Zitronensäure, oder Aminosäuren, wie z. B. Glycin, Alanin, Prolin und Arginin, und Peptide umgesetzt werden.

Als besonders bevorzugte organische Verbindungen werden gesättigte oder ungesättigte Carbonsäuren, Carbonsäureester, Carbonsäureanhydride, Aldehyde oder Lactone oder Gemische aus zwei oder mehr davon hydriert.

Beispiele dieser Verbindungen sind unter anderem Maleinsäure, Maleinsäureanhydrid, Bernsteinsäure, Bernsteinsäureanhydrid, Adipinsäure, 6-Hydroxycapronsäure, 2-Cyclododecylpropionsäure, die Ester der vorgenannten Säuren wie z. B. Methyl-, Ethyl-, Propyl- oder Butylester. Weitere Beispiele sind Hydroxypivalinaldehyd (HPA), Dimethylolbutanal (DMB), γ-Butyrolacton und Caprolacton.

Die zu hydrierende Carbonylverbindung kann dem Hydrierungsreaktor allein oder als Gemisch mit dem Produkt der Hydrierungsreaktion zugeführt werden, wobei dies in unverdünnter Form oder unter Verwendung von zusätzlichem Lösungsmittel geschehen kann. Als zusätzliches Lösungsmittel eigenen sich insbesondere Wasser, Alkohole wie Methanol, Ethanol und der Alkohol, der unter den Reaktionsbedingungen entsteht. Bevorzugte Lösungsmittel sind Wasser, THF und NMP, besonders bevorzugt ist Wasser.

Die Hydrierung sowohl in Sumpf- als auch in Rieselfahrweise, wobei jeweils bevorzugt in Kreislauffahrweise gearbeitet wird, führt man im allgemeinen bei einer Temperatur im Bereich von 50 bis 350 °C, bevorzugt im Bereich von 70 bis 300 °C, besonders bevorzugt im Bereich von 100 bis 270 °C und einem Druck im Bereich von 3 bis 350 bar, bevorzugt im Bereich von 5 bis 330 bar, besonders bevorzugt im Bereich von 10 bis 300 bar durch.

Die nach dem erfindungsgemäßen Verfahren hergestellten Katalysatoren können in Verfahren zur Herstellung von Hexandiol und/oder Caprolacton eingesetzt werden, wie sie in DE 196 07 954, DE 196 07 955, DE 196 47 348 und DE 196 47 349 beschrieben sind.

Unter Verwendung der erfindungsgemäß hergestellten Katalysatoren werden hohe Umsätze und Selektivitäten erzielt. Gleichzeitig weisen die Katalysatoren eine hohe chemische und mechanische Stabilität auf.

Geträgerte Hydrierkatalysatoren, wie sie erfindungsgemäß erhältlich sind, können zur Hydrierung von Carbonylgruppen aufweisenden organischen Verbindungen verwendet werden. Bevorzugt ist die organische Verbindung dabei eine Carbonsäure, ein Carbonsäureester, ein Carbonsäureanhydrid, ein Aldehyd oder ein Lacton.

Bevorzugt wird das Verfahren in Festbettreaktoren bei Wasserstoffdrücken um 200 bar und Temperaturen um 200 °C durchgeführt. Typischerweise werden Umsätze von mehr als 99,5 % mit Katalysatorbelastungen von maximal 0,1 bis 0,6 kg/(L*h) erreicht. Durch Einsatz der erfindungsgemäß hergestellten Katalysatoren kann die Katalysatorbelastung teilweise bis auf 1,8 kg/(L*h) verdreifacht werden, ohne dass ein Einbrechen des Umsatzes (Unterschreiten von 99,5 % Umsatz) zu beobachten ist. Die Selektivität sowie die mechanische Stabilität der Formkörper wurden dabei nicht negativ beeinflusst.

Die Erfindung wird nachstehend anhand von Beispielen näher erläutert.

### Beispiele

In den Beispielen wird ein Cu/Al₂O₃/La₂O₃-Katalysator gemäß WO 2007/006719, Beispiel 1, in Form von Tabletten der Dimension 1,5 x 1,5 mm oder 3 x 3 mm verwendet. Als Beispielreaktion wurde die Hydrierung von Adipinsäuredimethylester zu 1,6-Hexandiol gewählt. Zudem wurde auch die Hydrierung von Dimethylolbutanal (DMB) zu Trimethylolpropan untersucht.

Die mechanische Stabilität der Festkörperkatalysatoren wurde durch die Seitendruckfestigkeit bestimmt. Dabei wurde die Seitendruckfestigkeit mit einem Gerät des Typs Z 2.5/T 919 von Zwick Röll (Ulm) bestimmt.

### I. Präparation der Katalysatoren:

### Vergleichsbeispiel:

Die Herstellung entspricht Beispiel 1 aus WO 2007/0067.19 A1. Tablettiert wird unter Zusatz von 3 Gew.-% Graphit und 15 Gew.-% Cu-Blättchen zu 1.5 x 1.5 mm Tabletten. Der so hergestellte Katalysator enthält 56 Gew.-% Element Cu, 11.3 Gew.-% Element Al und 3.2 Gew.-% Element La. Die Seitendruckfestigkeit im oxidischen und unbenutzten Zustand beträgt durchschnittlich 67 N.

### Beispiel 1:

685 g Katalysatortabletten gemäß Vergleichsbeispiel werden während 48h mit 1000 ml NaOH 0.5 M bei Raumtemperatur behandelt. Die ausgebauten Katalysatortabletten werden anschließend mit destilliertem Wasser neutral gewaschen und 16 Stunden bei 120 °C getrocknet. Der so hergestellte Katalysator enthält 53 Gew.-% Element Cu, 10.3 Gew.-% Element Al und 3.1 Gew.-% Element La. Die Seitendruckfestigkeit im oxidischen und unbenutzten Zustand beträgt durchschnittlich 69 N.

### Beispiel 2:

1500 g Katalysatorpulver gemäß Vergleichsbeispiel (gleiche Vorschrift. ohne Vorkompaktierung und Tablettierung) werden mit 6000 ml NaOH 2.0 M bei 50 °C während 126 Stunden in Suspension gerührt. Die warme Suspension wird unverdünnt filtriert und mit kaltem destilliertem Wasser neutral gewaschen. Der Filterkuchen wurde 16 Stunden bei 120 °C getrocknet. Der trockene Filterkuchen wurde durch ein 0.8 mm Sieb passiert und mit 3 % Graphit und 15 % Cu-Blättchen zu 1.5 x 15 mm Tabletten gepresst. Die Tabletten wurden während 2 Stunden bei 350°C nachkalziniert. Der so hergestellte Katalysator enthält 59 Gew.-% Element Cu, 6.9 Gew.-% Element Al und 3.6 Gew.-% Element La. Die Seitendruckfestigkeit im oxidischen und unbenutzten Zustand beträgt durchschnittlich 46 N.

### Aktivierung:

Sämtliche Katalysatoren wurden mit einem N₂/H₂-Gemisch bei 1 bar und einer Temperatur von 180 °C in einer dem Fachmann geläufigen Prozedur reduziert.

### II. Hydrierung von Adipinsäuredimethylester zu 1,6-Hexandiol

### Vergleichsbeispiel

200 mL des gemäß Vergleichsbeispiel hergestellten Katalysators wurden aktiviert in einen ölmantelbeheizten Rohrreaktor (Durchmesser 14 mm) gefüllt. Bei 200 °C, 200 bar Wasserstoffdruck und einer Wasserstoffzufuhr von 200 NL/h wurde Adipinsäuredimethylester mit unterschiedlichen Zulaufraten kontinuierlich zudosiert. Der Reaktor wurde im einfachen Durchgang betrieben. Der Reaktor konnte in einem Belastungsbereich (WHSV) von bis zu 0,6 g/(mL*h) mit Esterumsätzen von > 99,5 % und 1,6-Hexandiol-Selektivitäten > 99,7 % betrieben werden.

| Belastung | Umsatz | Selektivität |
|---|---|---|
| g/(mL*h) | % | % |
| 0,3 | 99,86 | 99,20 |
| 0,4 | 99,82 | 99,51 |
| 0,6 | 99,58 | 99,70 |
| 0,7 | 99,35 | 99,77 |
| 0,8 | 98,68 | 99,77 |
| 0,9 | 98,26 | 99,80 |
| 1,0 | 97,00 | 99,81 |

### Beispiel 1

160 mL des gemäß Beispiel 1 hergestellten Katalysators wurden aktiviert in einen ölmantelbeheizten Rohrreaktor (Durchmesser 14 mm) gefüllt. Bei 200 °C, 200 bar Wasserstoffdruck und einer Wasserstoffzufuhr von 200 NL/h wurde Adipinsäuredimethylester mit unterschiedlichen Zulaufraten kontinuierlich zudosiert. Der Reaktor wurde im einfachen Durchgang betrieben. Der Reaktor konnte in einem Belastungsbereich (WHSV) bis zu 1,8 g/(mL*h) mit Esterumsätzen von > 99,7 % und 1,6-Hexandiol-Selektivitäten > 99,5% betrieben werden.

| Belastung g/(mL*h) | Umsatz % | Selektivität % |
|---|---|---|
| 0,4 | 99,86 | 98,93 |
| 0,7 | 99,85 | 99,32 |
| 1,1 | 99,84 | 99,60 |
| 1,5 | 99,82 | 99,69 |
| 1,8 | 99,69 | 99,71 |

### Beispiel 2

200 mL des gemäß Beispiel 2 hergestellten Katalysators wurden aktiviert in einen ölmantelbeheizten Rohrreaktor (Durchmesser 14 mm) gefüllt. Bei 200 °C, 200 bar Wasserstoffdruck und einer Wasserstoffzufuhr von 200 NL/h wurde Adipinsäuredimethylester mit unterschiedlichen Zulaufraten kontinuierlich zudosiert. Der Reaktor wurde im einfachen Durchgang betrieben. Der Reaktor konnte in einem Belastungsbereich (WHSV) bis zu 1,5 g/(mL*h) mit Esterumsätzen von > 99,8 % und 1,6-Hexandiol-Selektivitäten > 99,8 % betrieben werden.

| Belastung g/(mL*h) | Umsatz % | Selektivität % |
|---|---|---|
| 0,5 | 99,85 | 99,46 |
| 0,7 | 99,86 | 99,57 |
| 1,1 | 99,88 | 99,74 |
| 1,3 | 99,84 | 99,84 |
| 1,5 | 99,82 | 99,85 |

### III. Hydrierung von Dimethylolbutyraldehyd (DMB) zu Trimethylolpropan (TMP)

### Vergleichsbeispiel

150 mL des gemäß Vergleichsbeispiel hergestellten Katalysators wurden aktiviert und in einen ölbeheizten Doppelmantelrohrreaktor gefüllt. Bei 100 °C und 90 bar Wasserstoffdruck wurde eine 70 %ige wässrige DMB-Lösung mit unterschiedlichen Zulaufraten kontinuierlich zudosiert. Der Reaktor wurde mit einem Kreislauf zu Zulauf Verhältnis von 6:1 betrieben. Die Anlage konnte in einem Belastungsbereich (LHSV) bis zu 0.2 mL/(mL*h) mit Aldehydumsätzen von > 99,6% und TMP-Selektivitäten > 96 % betrieben werden.

| Belastung mL(mL*h) | Umsatz % | Selektivität % |
|---|---|---|
| 0,20 | 99,69 | 96,68 |
| 0,42 | 97,47 | 97,08 |
| 0,50 | 95,29 | 96,96 |

### Beispiel 1

150 mL des gemäß Beispiel 1 hergestellten Katalysators wurden aktiviert und in einen ölbeheizten Doppelmantelrohrreaktor gefüllt. Bei 100 °C und 90 bar Wasserstoffdruck wurde eine 70 %ige wässrige DMB-Lösung mit unterschiedlichen Zulaufraten kontinuierlich zudosiert. Der Reaktor wurde mit einem Kreislauf zu Zulauf Verhältnis von 6 : 1 betrieben. Der Reaktor konnte in einem Belastungsbereich (LHSV) bis zu 0.5 mL/(mL*h) mit Esterumsätzen von > 99,9% und TMP-Selektivitäten > 95 % betrieben werden.

| Belastung mL(mL*h) | Umsatz % | Selektivität % |
|---|---|---|
| 0,20 | 99,99 | 95,22 |
| 0,42 | 99,96 | 95,30 |
| 0,50 | 99,92 | 95,21 |

## Patentansprüche

1. Verfahren zur Herstellung eines geträgerten Hydrierkatalysators mit erhöhter Hydrieraktivität, der Kupfermetall und Kupferoxid auf einem Al₂O₃-haltigen Trägermaterial enthält, wobei der calcinierte geträgerte Hydrierkatalysator vor oder nach seiner endgültigen Formgebung sowie vor seinem Einsatz in der Hydrierung mit einer Basenlösung mit einem pH-Wert von > 10 bei einer Temperatur im Bereich von 20 bis 120 °C für 1 bis 300 Stunden behandelt wird, und wobei ein oxidisches Material, umfassend Kupferoxid und Aluminiumoxid und mindestens eines der Oxyde des Eisens, Lanthans, Wolframs, Molybdäns, Titans, Zirkoniums, Zinns oder Mangans bereitgestellt wird, dem oxidischen Material pulverförmiges metallisches Kupfer und/oder Kupferblättchen und optional zusätzlich pulverförmiger Zement, Graphit oder ein Gemisch davon zugegeben werden und das so resultierende Gemisch zu einem Formkörper verformt wird und wobei der Formkörper vor dem Einsatz als Katalysator durch Behandlung mit reduzierenden Medien aktiviert wird.

2. Verfahren nach Anspruch 1, **dadurch gekennzeichnet, dass** die Basenlösung ein Alkalimetall- oder Erdalkalimetallhydroxid oder ein Gemisch davon enthält.

3. Verfahren nach Anspruch 2, **dadurch gekennzeichnet, dass** die Basenlösung Natronlauge enthält.

4. Verfahren nach einem der Ansprüche 1 bis 3, **dadurch gekennzeichnet, dass** mit einer wässrigen Basenlösung behandelt wird.

## Claims

1. A process for preparing a supported hydrogenation catalyst with increased hydrogenation activity, which comprises copper metal and copper oxide on an Al₂O₃-containing support material, said calcined supported hydrogenation catalyst being treated before or after the final shaping thereof and before use thereof in the hydrogenation with a base solution having a pH of > 10 at a temperature in the range from 20 to 120°C for 1 to 300 hours, and wherein an oxidic material comprising copper oxide and aluminum oxide and at least one of the oxides of iron, lanthanum, tungsten, molybdenum, titanium, zirconium, tin or manganese is provided, pulverulent metallic copper and/or copper flakes and optionally additionally pulverulent cement, graphite or a mixture thereof is added to the oxidic material, and the resulting mixture is shaped to a shaped body and wherein the shaped body is activated prior to use as a catalyst by treatment with reducing media.

2. The process according to claim 1, wherein the base solution comprises an alkali metal hydroxide or alkaline earth metal hydroxide or a mixture thereof.

3. The process according to claim 2, wherein the base solution comprises sodium hydroxide.

4. The process according to any of claims 1 to 3, wherein treatment is effected with an aqueous base solution.

## Revendications

1. Procédé de fabrication d'un catalyseur d'hydrogénation à activité d'hydrogénation accrue, qui contient du cuivre métallique et de l'oxyde de cuivre sur un matériau support contenant de l'Al₂O₃, le catalyseur d'hydrogénation calciné supporté étant traité avant ou après sa mise en forme définitive ainsi qu'avant son utilisation dans l'hydrogénation avec une solution basique ayant une valeur de pH > 10 à une température dans une plage de 20 à 120°C pendant 1 à 300 heures, et un matériau de type oxyde comprenant de l'oxyde de cuivre et de l'oxyde d'aluminium et au moins l'un des oxydes de fer, de lanthane, de tungstène, de molybdène, de titane, de zirconium, d'étain ou de manganèse étant fourni, du cuivre métallique pulvérulent et/ou des paillettes de cuivre et en option en outre du ciment pulvérulent, du graphite ou un mélange de ceux-ci étant ajoutés au matériau de type oxyde et le mélange résultant étant façonné pour former un corps moulé et le corps moulé étant activé avant l'utilisation en tant que catalyseur par traitement avec des milieux réducteurs.

2. Procédé selon la revendication 1, **caractérisé en ce que** la solution basique contient un hydroxyde de métaux alcalins ou de métaux alcalino-terreux ou un mélange de ceux-ci.

3. Procédé selon la revendication 2, **caractérisé en ce que** la solution basique contient de la soude caustique.

4. Procédé selon l'une quelconque des revendications 1 à 3, **caractérisé en ce que** l'on effectue un traitement avec une solution basique aqueuse.
